# EUROPÄISCHE PATENTSCHRIFT

(11) **EP 1 781 252 B1**
(45) Veröffentlichungstag und Bekanntmachung des Hinweises auf die Patenterteilung: **09.01.2008**
(21) Anmeldenummer: 05774320.5
(22) Anmeldetag: 09.07.2005
(51) Int. Cl.: A61K 9/50

(54) **VERFAHREN ZUR HERSTELLUNG VON ÜBERZOGENEN ARZNEIFORMEN MIT STABILEM WIRKSTOFFFREIGABEPROFIL**
METHOD FOR PRODUCING COATED DRUGS HAVING A STABLE PROFILE FOR THE RELEASE OF ACTIVE INGREDIENTS
PROCEDES POUR PRODUIRE DES FORMES GALENIQUES ENROBEES A PROFIL STABLE DE LIBERATION DE PRINCIPE ACTIF

(30) Priorität: 23.07.2004 DE 102004035938
(43) Veröffentlichungstag der Anmeldung: 09.05.2007
(73) Patentinhaber: Evonik Röhm GmbH, 64293 Darmstadt (DE)
(72) Erfinder: PETEREIT, Hans-Ulrich, 64291 Darmstadt (DE); WEISBROD, Wolfgang, 64283 Darmstadt (DE); BÄR, Hans, 64753 Brombachtal (DE)
(86) Internationale Anmeldenummer: PCT/EP2005/007468
(87) Internationale Veröffentlichungsnummer: WO 2006/010457

(56) Entgegenhaltungen:
- EP-A- 0 553 392
- DE-A1- 19 835 823
- US-A- 4 728 513
- US-A- 5 639 476

## Beschreibung

Die Erfindung betrifft ein Verfahren zur Herstellung von Arzneiformen mit stabilem Wirkstofffreigabeprofil, wobei die Arzneiformen aufgrund eines Überzuges von Vinyl(co)polymeren eine kontrollierte Freisetzungscharakteristik aufweisen.

### Stand der Technik

US 4,600,645 beschreibt ein Verfahren zur Herstellung von überzogenen Arzneiformen mit stabilem Wirkstofffreigabeprofil. Dazu soll ein wirkstoffhaltiges Substrat mit einem die Wirkstofffreisetzung verzögernden Polymer überzogen werden und unmittelbar danach mit einem wasserlöslichen zweiten Überzug versehen werden. Die erhaltene Arzneiform soll dann für 15 bis 60 Minuten getrocknet werden. In einem Beispiel werden wirkstoffhaltige Pellets zunächst mit Ethylcellulose (Aquacoat^{®}) und anschließend mit Hydroxypropylmethylcellulose (HPMC) überzogen. Das Trocknen erfolgt direkt im für den Überzug verwendeten Wirbelschichtgerät z. B. bei 70 °C für 30 Minuten und bei Rotorgeschwindigkeit von 100 Upm. Überzogene wirkstoffhaltige Pellets, die bei 58 °C für 60 Minuten getrocknet wurden, weisen in etwa das gleiche Freisetzungsprofil auf, wie bei einer Trocknung für 60 °C für eine Woche in einem beheizten Schrank.

EP 0 553 392 A1 beschreibt Arzneiformen mit aus wäßrigen Dispersionen hergestellten Acrylpolymerüberzügen mit stabilisiertem Wirkstofffreigabeprofil. Die Überzüge können z. B. aus Polymeren von Typ EUDRAGIT^{®} RL/RS bestehen. Ein stabiles Wirkstofffreigabeprofil wird erreicht, indem die überzogenen Arzneiformen in einem beheizten Schrank bei einer Temperatur um die 45 °C für 24 bis 48 Stunden konditioniert werden.

US 5,273,760 beschreibt mit Ethylcellulose aus wäßriger Dispersion überzogene Arzneiformen mit stabilisiertem Wirkstofffreigabeprofil. Dabei wird durch eine mehrtägige Lagerung, z. B. für 72 Stunden, bei 60 °C und 60 - 80 % Leuchtfeuchte in etwa derselbe Effekt erreicht, wie mit einmonatiger Lagerung bei 37 °C und 80 % Luftfeuchte.

### Aufgabe und Lösung

Arzneiformen, die aufgrund eines Überzuges von Vinyl(co)polymeren eine kontrollierte Freisetzungscharakteristik entfalten, weisen unmittelbar nach dem Aufbringen der Sprühsuspensionen noch kein reproduzierbares Freisetzungsprofil auf. Erfahrungsgemäß ist dieser Effekt bei Überzügen, die aus wässrigen Dispersionen hergestellt wurden am ausgeprägtesten, kommt jedoch auch bei Überzügen, die aus organischen Lösungen aufbracht wurden zum Tragen. Oftmals empfehlen die Hersteller entsprechender Vinyl(co)polymere ihren Kunden, die damit Arzneiformen herstellen, nach dem Auftragen der Überzuge, eine längere Trocknungsphase für z. B. 16 bis 48 Stunden anzuschließen, um stabile Wirkstofffreigabeprofile zu erhalten. Diese Vorgehensweise hat sich in der Praxis bewährt, ist jedoch insbesondere bei großen Arzneimittelchargen aufwendig.

Die EP 0 553 392 A1 schlägt, ähnlich wie die oben genannten Empfehlungen der Hersteller, zur Erreichung eines reproduzierbaren Freisetzungsprofils von Arzneiform mit aus wäßrigen Dispersionen hergestellten Acrylpolymerüberzügen eine Konditionierung in einem beheizten Schrank bei einer Temperatur um die 45 °C für 24 bis 48 Stunden vor. Dieses Verfahren hat sich in der Praxis bewährt, ist jedoch insbesondere bei großen Arzneimittelchargen aufwendig.

US 4,600,645 beschreibt ein Konditionierungsverfahren, bei dem die Arzneiformen für 15 bis 60 Minuten getrocknet werden sollen. Die Erfinder haben jedoch festgestellt, daß sich dieses Verfahren offenbar nur für Überzüge auf Cellulosebasis und nicht für mit Vinyl(co)polymeren überzogene Arzneiformen eignet. Überzüge aus Vinyl(co)polymeren können bei einer Konditionierung gemäß der US 4,600,645 leicht rissig werden. Die unter dem Mikroskop sichtbaren Risse führen zu einer unkontrollierten Wirkstofffreisetzung und verhindern das Erreichen eines stabilisierten Zustandes von vorn herein.

Es wurde als eine Aufgabe gesehen, ein Verfahren für Arzneiformen, die aufgrund eines Überzuges von Vinyl(co)polymeren eine kontrollierte Freisetzungscharakteristik aufweisen, bereitzustellen, das es ermöglicht in kurzer Zeit und mit wenig Aufwand zu stabilen Wirkstofffreigabeprofilen zu gelangen.

### Die Aufgabe wird gelöst durch ein

Verfahren zur Herstellung von Arzneiformen mit stabilem Wirkstofffreigabeprofil, wobei die Arzneiformen aufgrund eines Überzuges von Vinyl(co)polymeren eine kontrollierte Freisetzungscharakteristik aufweisen,
dadurch gekennzeichnet, daß
die überzogenen Arzneiformen in einem Wirbelschicht-Überzugsgerät oder einem Trommel-Überzugsgerät für mindestens 10 Minuten bis zum Erreichen eines stabilen Wirkstofffreigabeprofils bei einer Temperatur von 30 bis 70 °C konditioniert werden, wobei während der Konditionierung eine Luftfeuchte von 5 bis 30 % eingestellt wird.

### Ausführung der Erfindung

Die Erfindung betrifft ein Verfahren zur Herstellung von Arzneiformen, die aufgrund eines Überzuges von Vinyl(co)polymeren eine kontrollierte Freisetzungscharakteristik aufweisen, mit stabilem Freigabeprofil.

### Kontrollierte Freisetzungscharakteristik

Der Begriff "kontrollierte" Freisetzungscharakteristik ist dem Fachmann bekannt. Der Fachmann versteht darunter z. B., dass die Freisetzungsprofile für bestimmte Wirkstoffe mittels der Formulierung der Arzneiform, insbesondere durch die Wahl des äußeren Überzugs, in reproduzierbarer Weise an den Wirkstoff angepasst werden. Bekannte "kontrollierte" Freisetzungscharakteristiken sind die pH-gesteuerte Wirkstofffreisetzung oder die verzögerte, auch als retardiert bezeichnete Wirkstofffreisetzung. Nach der Freigabe der Arzneiform zum Verkauf darf sich das Freigabeprofil auch nach längerer Lagerung nicht mehr wesentlich verändern, um die therapeutische Wirkung in-vivo sicherzustellen. Standardisierte Methoden zur Bestimmung des Freigabeprofils sind dem Fachmann hinlänglich bekannt. Die USP27 - NF22 Supplement 1 <711> Dissolution und <724> Drug Release beschreiben Methoden zur Bestimmung des Freigabeprofils und zulässige Toleranzen.

Überzuge aus Vinyl(co)polymeren, die Arzneiformen eine kontrollierte Freisetzungscharakteristik verleihen, sind hinlänglich bekannt. Zu nennen sind insbesondere Überzüge aus Vinyl(co)polymere von Typ der (Meth)acrylat(co)polymere oder von Typ der Polyvinylacetate einschließlich den Derivaten des Polyvinylacetats.

Überzüge aus (Meth)acrylatcopolymeren mit neutralen Resten, (Meth)acrylatcopolymeren mit funktionellen quaternären Aminogruppen, sowie Polyvinylacetate bewirken durch ihren pH-unabhängig, im Darmsaft langsam quellenden Charakter eine retardierende, kontrollierte Wirkstofffreisetzung. Überzüge aus (Meth)acrylatcopolymeren mit anionischen funktionellen Gruppen bewirken eine durch das alkalische Millieu des Darms pH-gesteuerte, kontrollierte Wirkstofffreisetzung. Überzüge aus (Meth)acrylatcopolymere mit funktionellen tertiären Aminogruppen dienen der Geschmacksisolierung und bewirken durch das saure Millieu des Magens pH-gesteuerte, rasche kontrollierte Wirkstofffreisetzung.

Das Verfahren eignet sich insbesondere für Arzneiformen mit Überzügen aus Vinyl(co)polymeren, die aus wässrigen Dispersionen aufgebracht wurden. Das Verfahren eignet sich weiterhin insbesondere für Arzneiformen mit Überzügen, die eine retardierende Wirkstofffreisetzung bewirken.

### Stabiles Wirkstofffreigabeprofil

Im Sinne der USP27 - NF22 Supplement 1 <711> Dissolution und <724> Drug Release können Abweichung von mehr als +/-10 % der für die jeweilige Arzneiform bzw. Einzeldosis deklarierten Wirkstoffmenge als eine wesentliche, nicht tolerierbare Veränderung angesehen werden, die in-vivo zu einer veränderten therapeutischen Wirkung führen können. In diesem Fall könnte man die Arzneiform als instabil bezeichnen. Der Begriff "stabiles Wirkstofffreigabeprofil" wird daher so definiert, wie ihn ein Fachmann unter Berücksichtigung der USP - NF versteht.

Unter einem stabilen Wirkstofffreigabeprofil im Sinne der Erfindung wird ein Wirkstofffreigabeprofil verstanden, das sich gegenüber einem Wirkstofffreigabeprofil einer Referenzpräparation, die für 24 Stunden bei 40 °C im Umlufttrockenschrank konditioniert wurde um nicht mehr als, +/-10 %, unterscheidet. Die %-Angabe bezieht sich dabei auf den für die jeweilige Arzneiform bei der Zulassungsbehörde (z. B. EMEA in Europa oder FDA in den USA) deklarierten Wirkstoffgehalt der Einzeldosis und den deklarierten Messzeitpunkten.

Beispielsweise kann für eine Arzneiform "X" in Tablettenform für eine Tablette die Wirkstoffmenge "Y" deklariert sein. Zu den Zeitpunkten "Z₁" und "Z₂" und "Z₃" müssen dann unter definierten Bedingungen jeweils spezifizierte Teilmengen "Ti", "T₂" und "T₃" der Wirkstoffmenge "Y" freigesetzt werden. Zulässige Abweichungen zu den angegebenen Zeitpunkten betragen ausgehend vom jeweiligen Sollwert der angegebenen Teilmengen "T₁", "T₂" und "T₃" nicht mehr als +/- 10 % der Wirkstoffmenge "Y". Ist z. B. der Wert "T₂" 50 %, so sind Werte im Bereich von 40 bis 60 % als stabil anzusehen.

Der Referenzwert entspricht dabei dem in EP 0 553 392 A1 vorgeschlagenen Verfahren, mit dem stabilen Freisetzungsprofile bei Arzneiformen, die aufgrund eines Überzuges von bestimmten Vinyl(co)polymeren eine kontrollierte Freisetzungscharakteristik aufweisen, erreicht werden. Die Erfinder haben festgestellt, daß sich das Verfahren der EP 0 553 392 A1 allgemein als Referenzwert für Vinyl(co)polymeren mit kontrollierter Freisetzungscharakteristik eignet.

Als Wirkstofffreisetzungskurve oder Freisetzungscharakteristik ist dabei die, für die jeweilige Arzneiform in Bezug auf geforderte therapeutische Wirkung geforderte bzw. relevante Wirkstofffreisetzungskurve bzw. Freisetzungscharakteristik, zugrunde zu legen. Diese ist praktisch für jede Arzneiform, je nach enthaltenem Wirkstoff und Polymerüberzugstyp unterschiedlich.

Die Wirkstofffreisetzungskurve einer Arzneiform mit einem retardierenden Polymerüberzugstyp kann z. B. über 4 bis 12 Stunden bei einem konstantem pH-Wert aufgenommen werden. Die Wirkstofffreisetzungskurve einer Arzneiform mit einem anionischen Polymerüberzugstyp wird in der Regel erst für 2 Stunden bei pH 1,2, zum Test der Magensaftresistenz, und anschließend für mehrere bei einem konstantem höheren pH-Wert, zum Test der Wirkstofffreisetzung, aufgenommen. Die Freisetzungskurve einer Arzneiform mit einem kationischen Polymerüberzugstyp mit tertiären Aminogruppen kann z. B. bei einem konstantem pH-Wert über einen kurzen Zeitraum von 10 bis 60 mindestens aufgenommen werden, da sich diese Überzugstypen vergleichsweise schnell auflösen.

### Die Herstellung von mit Vinyl(co)polymeren überzogenen Arzneiformen

Wirkstoffhaltige Kerne bzw. Pelletkerne bilden in an sich bekannter Weise die Grundlage für die Überzüge aus Vinyl(co)polymeren. Die Pelletierung kann auf wirkstofffreie Kugeln (Nonpareilles) erfolgen oder es können kernfreie Pellets, Pelletkerne, erzeugt werden. Zunächst wird ein ausgerundetes, wirkstoffhaltiges Substrat mit oder ohne Kern erzeugt. Mittels eines Wirbelschichtverfahrens kann Flüssigkeit auf Placebopellets oder sonstige geeignete Trägermaterialien aufgebracht werden, wobei das Lösungs- oder Suspendiermittel verdunstet wird. Nach dem Herstellverfahren kann sich ein Trocknungsschritt anschließen. Die noch nicht überzogene, ausgerundete Schicht wird je nach Größe z. B. als Kern oder Pelletkern bezeichnet.

Der Wirkstoff wird in der Regel in einem organischen Lösemittel oder in Wasser eingetragen und vermischt. Um eine befriedigende Sprühbarkeit des Gemisches zu gewährleisten, ist es meist notwendig, ein Gemisch mit niedriger Viscosität zu formulieren. Der Zusatz eines Detergenz, z. B. Tween in Konzentrationen von 0,1 bis 20, bevorzugt 0,5 bis 10 Gew.-% kann zur Herabsetzung der Oberflächenspannung vorteilhaft sein.

Neben dem Wirkstoff können sie weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Entsprechende Auftragsverfahren sind z. B. aus Bauer, Lehmann, Osterwald, Rothgang, "Überzogene Arzneiformen" Wissenschaftliche Verlagsgesellschaft mbH Stuttgart, Kap 7, S.165 - 196, bekannt.

Details sind dem Fachmann weiterhin aus Lehrbüchern bekannt. Siehe z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

Pelletkerne können durch Verfahren wie Rotagglomeration, Ausfällen oder Sprühverfahren, insbesondere Ultraschall-Wirbel-Sprühverfahren, zu noch nicht überzogenen Kernen oder Pelletkernen definierter Größe, z. B. 50 bis 1000 *µ*m, ausgerundet werden. Dies hat den Vorteil, daß das gesamte Kernvolumen zur Wirkstoffbeladung zur Verfügung steht. Die Wirkstoffbeladung kann dadurch gegenüber der Ausführungsform mit inertem Kern nochmals erhöht werden.

Nach Herstellung der wirkstoffhaltigen Kerne oder Pelletkerne werden diese im Sprühverfahren mit einem äußeren Überzug aus Vinyl(co)polymeren versehen, so dass man überzogene Pellets erhält. Die Herstellung der Pellets erfolgt mittels Sprühauftrag aus organischer Lösung, oder bevorzugt aus wäßrigen Dispersionen. Für die Ausführung ist dabei entscheidend, daß gleichmäßige, porenfreie Überzüge entstehen. In der Regel werden die überzogenen Pellets nach dem Sprühauftrag noch für einige Minuten nachgetrocknet, bevor mit dem Konditionierungverfahren begonnen wird. In der Regel enthalten die Polymerüberzüge pharmazeutisch übliche Hilfsstoffe wie z. B. Trennmittel oder Weichmacher.

### Das Konditionierungsverfahren

Die überzogenen Arzneiformen werden in einem Wirbelschicht-Überzugsgerät (Wirbelschicht-Coater) oder einem Trommel-Überzugsgerät (Trommel-Coater) für mindestens 10 Minuten bis zum Erreichen eines stabilen Wirkstofffreigabeprofils Konditioniert. Diese Bedingung ist in der Regel bei einer Trocknungszeit von 10 bis 120, 15 bis 90, insbesondere 15 bis 60 Minuten erfüllt. Die Trocknungstemperatur beträgt 30 bis 70, bevorzugt 35 bis 65, besonders bevorzugt 40 bis 60 °C.

Dem Fachmann aus der Galenik sind die Gerätschaften, das Wirbelschicht-Überzugsgerät (Wirbelschicht-Coater) bzw. das Trommel-Überzugsgerät (Trommel-Coater) bekannt. Im Wirbelschicht-Überzugsgerät können wirkstoffhaltige Kerne oder wirkstoffhaltige Pellets z. B. mit einem Überzug aus Vinyl(co)polymeren versehen werden. Zu diesem Zweck werden die wirkstoffhaltige Kerne oder wirkstoffhaltige Pellets in einem Luftstrom zu einer permanenten Auf- und Abbewegung verwirbelt, während man gleichzeitig das (Meth)acrylatcopolymer in Form einer fein vernebelten Dispersion einsprüht. Die Polymerdispersion schlägt sich auf den wirkstoffhaltigen Kerne oder wirkstoffhaltige Pellets nieder und verfilmt dort. Das enthaltene Wasser verdunstet im Luftstrom, bei dem in Regel eine Temperatur im Bereich von 30 bis 70 °C eingestellt ist. Trommel-Überzugsgerät (Trommel-Coater) erfolgt die Bewegung der wirkstoffhaltigen Kerne oder wirkstoffhaltige Pellets durch die Trommelbewegung.

Konditioniert man wesentlich länger als notwendig, wenn man die Anlage beispielsweise über Nacht laufen lässt, kann es durch Abrieb zu mechanischen Beeinträchtigungen kommen, wodurch das zunächst erreichte stabile Wirkstofffreigabeprofil wieder verloren geht. Man trocknet daher nur bis zum Erreichen des stabilen Wirkstofffreigabeprofils, bzw. bis in den Bereich eines stabilen Wirkstofffreigabeprofils hinein, jedoch nicht bzw. nicht wesentlich darüber hinaus.

Während der Trocknung wird eine Luftfeuchte von 5 bis 30, bevorzugt 10 bis 25 % eingestellt. Die Luftfeuchte bezieht sich auf die jeweilige Trocknungstemperatur und kann z. B. hinter der Filtereinheit im der Abluft des Wirbeischicht-Coating-Geräts mit entsprechenden Sensoren gemessen bzw. festgestellt werden. Die erforderliche Luftfeuchte kann zweckmäßigerweise durch Einsprühen von Wasser herstellt werden.

Zur Vermeidung von Klebrigkeit und zur Erhöhung der mechanischen Festigkeit kann man vorteilhafterweise eine wässrige Suspension, enthaltend 0,1 bis 5, bevorzugt 0,2 bis 1 Gew.-% eines Trennmittels, z. B. Talkum, Magnesiumstearat oder eine Kieselsäure (z. B. von Typ Syloid^{®}), bezogen auf die Suspension, einsprühen.
Das Verfahren kann bevorzugt sehr effizient ausgeführt werden, wenn die überzogenen Arzneiformen in einem ersten Schritt überzogene Arzneiformen durch Überziehen von wirkstoffhaltigen Kernen oder Pellets mit (Meth)acrylatcopolymeren im Wirbelschicht-Überzugsgerät oder einem Trommel-Überzugsgerät hergestellt werden und in einen zweiten Schritt die Konditionierung der überzogenen Arzneiformen im selben Gerät unmittelbar auf die Herstellung erfolgt.

Die erhaltenen konditionierten Pellets können bevorzugt mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften weiterverarbeitet werden.

### Überzüge aus neutralen Vinyl(co)polymeren

### a) Neutrale (Meth)acrylatcopolymere, Typ EUDRAGIT^{®} NE

Das erfindungsgemäße Verfahren eignet sich für Arzneiformen, deren Überzüge aus (Meth)acrylat(co)polymeren bestehen, die zu mehr als 95 Gew.-% bis 100 % aus Monomeren mit neutralen Resten polymerisiert sind. Monomere mit neutralen Resten können insbesondere C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat sein.

Zu nennen sind z. B. neutrale (Meth)acrylat Copolymere aus 20 bis 40 Gew.-% Ethylacrylat und 60 bis 80 Gew.-% Methylmethacrylat (Typ EUDRAGIT® NE). Gegebenenfalls können die genannten weitgehend neutralen (Meth)acrylatcopolymer geringe Anteile, z. B. 0 bis weniger als 5, bevorzugt 0 bis 2 Gew.-% an (Meth)acrylat-Monomeren mit einer anionischen Gruppe im Alkylrest kann z. B. Acrylsäure, bevorzugt jedoch Methacrylsäure, enthalten. Das überwiegend oder völlig neutrale Copolymer hat bevorzugt die Eigenschaft oberhalb von pH 5,0 in Wasser bzw. im Darmsaftmillieu zu quellen und Wirkstoff freizusetzen.

EUDRAGIT^{®} NE ist ein Copolymer aus 30 Gew.-% Ethylacrylat und 70 Gew.-% Methylmethacrylat. Das Polymer kann z. B. in Form einer 30 %-igen wäßrigen Dispersion, EUDRAGIT^{®} NE 30D, eingesetzt werden.

### b) Polyvinylacetate

Der innere Überzug kann ein (Co)polymer enthalten, das Polyvinylacetat bzw. ein Polyvinylacetat ist. Der Begriff "ein Polyvinylacetat" schließt Derivate des Polyvinylacetats mit ein. Das Polyvinylacetat kann als Dispersion vorliegen (z. B. vom Typ Kollicoat^{®} SR 30 D, Hersteller BASF, Polyvinylacetat Dispersion, stabilisiert mit Povidon und Na-Laurylsulfat).

### (Meth)acrylatcopolymer mit funktionellen anionischen Resten

Das Verfahren eignet sich für Arzneiformen mit Überzügen aus (Meth)acrylatcopolymeren, die zu 25 bis 95 Gew.-% aus radikalisch polymerisierten C₁- bis C₄-Alkylestern der Acryl- oder der Methacrylsäure und zu 5 bis 75 Gew.-% aus (Meth)acrylat-Monomere mit einer anionischen Gruppe im Alkylrest bestehen.

In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein.

### Überzugstypen EUDRAGIT^{®} L, L100-55, S und FS

Das Verfahren eignet sich für Arzneiformen mit Überzügen aus (Meth)acrylatcopolymeren aus 40 bis 60, Gew.-% Methacrylsäure und 60 bis 40 Gew.-% Methylmethacrylat oder 60 bis 40 Gew.-% Ethylacrylat (Typen EUDRAGIT^{®} L oder EUDRAGIT^{®} L100-55).

EUDRAGIT^{®} L ist ein Copolymer aus 50 Gew.-% Methylmethacrylat und 50 Gew.-% Methacrylsäure. EUDRAGIT^{®} L 30D ist eine Dispersion enthaltend 30 Gew.-% EUDRAGIT^{®} L.

EUDRAGIT^{®} L100-55 ist ein Copolymer aus 50 Gew.-% Ethylacrylat und 50 Gew.-% Methacrylsäure. EUDRAGIT^{®} L 30-55 ist eine Dispersion enthaltend 30 Gew.-% EUDRAGIT^{®} L 100-55.

Das Verfahren eignet sich für Arzneiformen mit Überzügen aus (Meth)acrylatcopolymeren aus aus 20 bis 40 Gew.-% Methacrylsäure und 80 bis 60 Gew.-% Methylmethacrylat (Typ EUDRAGIT^{®} S).

Das Verfahren eignet sich für Arzneiformen mit Überzügen aus (Meth)acrylatcopolymeren aus 10 bis 30 Gew.-%, Methylmethacrylat, 50 bis 70 Gew.-% Methylacrylat und 5 bis 15 Gew.-% Methacrylsäure (Typ EUDRAGIT^{®} FS).

EUDRAGIT^{®} FS ist ein Copolymer aus 25 Gew.-%, Methylmethacrylat, 65 Gew.-% Methylacrylat und 10 Gew.-% Methacrylsäure. EUDRAGIT^{®} FS 30 D ist eine Dispersion enthaltend 30 Gew.-% EUDRAGIT^{®} FS.

### Überzugstypen EUDRAGIT^{®} mit mittlerem Gehalt an Methacrylsäure

Das Verfahren eignet sich für Arzneiformen mit Überzügen aus (Meth)acrylatcopolymeren aus anionische (Meth)acrylat Copolymere aus 20 bis 34 Gew.-% Methacrylsäure und/oder Acrylsäure, 20 bis 69 Gew.-% Methylacrylat und 0 bis 40 Gew.-% Ethylacrylat und gegebenenfalls 0 bis 10 Gew.-% weiteren vinylisch copolymerisierbarern Monomeren, mit der Maßgabe, daß die Glastemperatur des Copolymers nach ISO 11357-2, Punkt 3.3.3, höchstens 60 °C beträgt. (Typ EUDRAGIT^{®} mit mittlerem Gehalt an Methacrylsäure).

Das Copolymer setzt sich insbesondere zusammen aus radikalisch polymerisierten Einheiten von
20 bis 34, bevorzugt 25 bis 33, besonders bevorzugt 28 bis 32 Gew.-% Methacrylsäure oder Acrylsäure, bevorzugt ist Methacrylsäure,
20 bis 69, bevorzugt 35 bis 65, besonders bevorzugt 35 bis 55 Gew.-% Methylacrylat und gegebenenfalls
0 bis 40, bevorzugt 5 bis 35, besonders bevorzugt 15 bis 35 Gew.-% Ethylacrylat zusammen, mit der Maßgabe, daß die Glastemperatur des Copolymers (ohne Weichmacherzusatz) nach ISO 11357-2, Punkt 3.3.3, höchstens 60, bevorzugt 40 bis 60, besonders bevorzugt 45 bis 55 °C beträgt.

Das (Meth)acrylat-Copolymer kann im wesentlichen bis ausschließlich aus den Monomeren Methacrylsäure, Methylacrylat und Ethylacrylat in den oben angegebenen Mengenanteilen bestehen. In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

### (Meth)acrylatcopolymer mit funktionellen kationischen Resten

### a) (Meth)acrylatcopolymere mit tertiären Aminogruppen, Typ EUDRAGIT® E100 und EPO

Das Verfahren eignet sich für Arzneiformen mit Überzügen aus (Meth)acrylatcopolymeren aus 30 bis 80 Gew.-% radikalisch polymerisierten C₁-bis C₄-Alkylestern der Acryl- oder der Methacrylsäure und 70 bis 20 Gew.-% (Meth)acrylat-Monomeren mit einer tertiären Aminogruppe im Alkylrest.

Geeignete Monomere mit funktionellen tertiären Aminogruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Der Gehalt der Monomere mit tertiären Aminogruppen im Copolymeren kann zwischen 20 und 70 Gew.-%, bevorzugt zwischen 40 und 60 Gew.-% liegen. Der Anteile der C₁- bis C₄-Alkylestern der Acryl- oder Methacrylsäure beträgt 70 - 30 Gew.-%. Zu nennen sind Methylmethacrylat, Ethylmethacrylat, Butylmethacrylat, Methylacrylat, Ethylacrylat und Butylacrylat.

Ein gebräuchliches (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein.

Ein handelsübliches (Meth)acrylatcopolymer mit tertiären Aminogruppen ist z. B. aus 25 Gew.-% Methylmethacrylat, 25 Gew.-% Butylmethacrylat und 50 Gew.-% Dimethylaminoethylmethacrylat aufgebaut (EUDRAGIT^{®} E100).

Ein weiteres handelsübliches (Meth)acrylatcopolymer mit tertiären Aminogruppen ist z. B. EUDRAGIT^{®} E PO: Copolymer aus Methylmethacrylat, Butylmethacrylat, und Dimethylaminoethylmethacrylat in Verhältnis 25 : 25 : 50 mit einer mittleren Teilchengröße von 15 µm.

### b) (Meth)acrylatcopolymere mit quaternären Aminogruppen, Typ EUDRAGIT^{®} RS oder RL

Das Verfahren eignet sich insbesondere für Arzneiformen mit Überzügen aus (Meth)acrylatcopolymeren mit quaternären Ammoniumgruppen, insbesondere aus Copolymeren, die aus radikalisch polymerisierten Einheiten von 50 - 70 Gew.-% Methylmethacrylat, 20 - 40 Gew.-% Ethylacrylat und 12 - 2 Gew.-% 2-Trimethylammoniumethylmethacrylat-Chlorid aufgebaut sind (Typ EUDRAGIT^{®} RS oder RL). Das Verfahren eignet sich insbesondere für Arzneiformen mit Überzügen aus (Meth)acrylatcopolymeren mit quaternären Ammoniumgruppen, die aus wässrigen Dispersionen aufgebracht wurden.

Das Verfahren eignet sich insbesondere für Arzneiformen mit Überzügen aus (Meth)acrylatcopolymeren, die aus radikalisch polymerisierten Einheiten von 65 Gew.-% Methylmethacrylat, 30 Gew.-% Ethylacrylat und 5 Gew.-% 2-Trimethylammoniumethylmethacrylat-Chlorid aufgebaut sind (EUDRAGIT^{®} RS).

Das Verfahren eignet sich insbesondere für Arzneiformen mit Überzügen aus (Meth)acrylatcopolymeren, die aus radikalisch polymerisierten Einheiten von 60 Gew.-% Methylmethacrylat, 30 Gew.-% Ethylacrylat und 10 Gew.-% 2-Trimethylammoniumethlymethacrylat-Chlorid (EUDRAGIT^{®} RL).

Das Verfahren eignet sich insbesondere für Arzneiformen mit Überzügen, die aus Mischungen von EUDRAGIT^{®} RS und EUDRAGIT^{®} RL bestehen.

### Wirkstoffe

Das erfindungsgemässe Verfahren eignet sich für im Prinzip für alle mit Vinyl(co)polymeren überzogenen Arzneiformen. Die Arzneiformen können beispielsweise folgende Wirkstoffe, Wirkstoffklassen bzw. Wirkstofftypen enthalten.

Gebräuchliche Arzneistoffe sind in Nachschlagewerken, wie z.B. der Roten Liste oder dem Merck Index zu entnehmen.

### Biologisch aktive Substanzen:

*Arzneistoffe sind dazu bestimmt, am oder im menschlichen oder tierischen Körper Anwendung zu finden, um*
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

### Therapieklassen

Diese pharmazeutisch aktiven Substanzen können einer oder mehrerer Wirkstoffklassen angehören, wie ACE-Hemmer, Adrenergika, Adrenocortikosteroide, Aknetherapeutika, Aldose-Reduktase-Hemmer, Aldosteron-Antagonisten, Alpha-Glucosidasehemmer, Alpha 1- Antagonisten, Mittel gegen Alkoholabusus, Aminosäuren, Amöbizide, Anabolika, Analeptika, Anaesthetika-Zusätze, Anaesthetika (nicht inhalativ), Anaesthetika (lokal), Analgetika, Androgene, Anginatherapeutika, Antagonisten, Antiallergika, Antiallergika wie PDE-Hemmer, Antiallergika zur Asthmabehandlung, Weitere Antiallergika (z.B. Leukotrienantagonisten, Antianämika, Antiandrogene, Antianxiolytika, Antiarthritika, Antiarrhythmika, Antiatheriosklerotika, Antibiotika, Anticholinergika, Anticonvulsiva, Antidepressiva, Antidiabetika, Antidiarrhoika, Antidiuretika, Antidots, Antiemetika, Antiepileptika, Antifibrinolytika, Antiepileptika, Antihelmintika, Antihistaminika, Antihypotensiva, Antihypertensiva, Antihypertonika, Antihypotonika, Antikoagulantien, Antimykotika, Antiöstrogene, Antiöstrogene (Nicht-Steroide), Antiparkinson-Mittel, Antiphlogistika, Antiproliferative Wirkstoffe, Antiprotozoen Wirkstoffe, Antirheumatika, Antischistosomizide, Antispasmolytika, Antithrombotika, Antitussiva, Appetitzügler, Arteriosklerosemittel, Bakteriostatika, Betablocker, Betarezeptorenblocker, Bronchodilatoren, Carboanhydrase-Hemmer, Chemotherapeutika, Choleretika, Cholinergika, Cholinergische Agonisten, Cholinesterase-Hemmer, Mittel zur Behandlung von Colitis ulcerosa, Cyclooxigenasehemmer, Diuretika, Ektoparasitizide, Emetika, Enzyme, Enzym-Hemmer, Enzyminhibitoren, Wirkstoffe gegen Erbrechen, Fibrinolytika, Fungistatika, Gabapentin, Gichtmittel, Glaukomtherapeutika, Glucocorticoide, Glucocortikosteroide, Hämostatika, Herzglykoside, Histamin H2-Antagonisten, Hormone und deren Hemmstoffe, Immuntherapeutika, Kardiotonika, Kokkidiostatika, Laxantien, Lipidsenker, Magen-Darmtherapeutika, Malariatherapeutika, Migränemittel, Mikrobiozide, Morbus Crohn, Metastasenhemmer, Migränemittel, Mineralstoffpräparate, motilitätssteigernde Wirkstoffe, Muskelrelaxantien, Neuroleptika, Wirkstoffe zur Behandlung der Oestrogene, Osteoporose, Otologika, Parkinsonmittel, Phytopharmaka, Pitavastatin, Protonenpumpenhemmer, Prostaglandine, Wirkstoffe zur Behandlung der benignen Prostatahyperblasie, Wirkstoffe zur Behandlung des Pruritus, Psoriasis Wirkstoffe, Psychopharmaka, Radikalfänger, Renin-Antagonisten, Schilddrüsentherapeutika, Wirkstoffe zur Behandlung von Seborrhoe, Wirkstoffe gegen Seekrankheit, Spasmolytika, alpha- und beta-Sympatomimetika, Tenatoprazol, Thrombozytenaggregationshemmer, Tyrosinkinaseinhibitoren, Tranquilizer, Ulkustherapeutika, Weitere Ulkustherapeutika, Mittel zur Behandlung der Urolithiasis, Virustatika, Virustatika, Vitamine, Zytokine, Wirkstoffe für die Kombinationstherapie mit Zytostatika, Zytostatika.

### Wirkstoffe

Beispiele geeigneter Wirkstoffe sind Acarbose, Acetylsalicylsäure, Abacavir, Aceclofenac, Aclarubicin, Acyclovir, Actinomycin, Adalimumab, Adefovir, Adefovirdipivoxil, Adenosylmethionin, Adrenalin und Adrenalinderivate, Agalsidase alpha, Agalsidase beta, Alemtuzumab, Almotriptan, Alphacept, Allopurinol, Almotriptan, Alosetron, Alprostadil, Amantadin, Ambroxol, Amisulprid, Amlodipin, Amoxicillin, 5-Aminosalicylsäure, Amitriptylin, Amlodipin, Amoxicillin, Amprenavir, Anagrelid, Anakinra, Anastrozol, Androgen und Androgenderivate, Apomorphin, Aripiprazol, Arsentrioxid, Artemether, Atenolol, Atorvastatin, Atosiban, Azathioprin, Azelainsäure, Barbitursäurederivate, Balsalazid, Basiliximab, Beclapermin, Beclomethason, Bemiparin, Benzodiazepine, Betahistin, Bexaroten. Bezafibrat, Bicalutamid, Bimatoprost, Bosentan, Botulinumtoxim, Brimonidin, Brinzolamid, Budesonid, Budipin, Bufexamac, Bumetanid, Buprenorphin, Bupropion, Butizin, Calcitonin, Calciumantagonisten, Calciumsalze, Candesartan, Capecitabin, Captopril, Carbamazepin, Carifenacin, Carvedilol, Caspofungin, Cefaclor, Cefadroxil, Cefalexin Cefalosporine, Cefditoren, Cefprozil, Cefuroxim, Celecoxib, Cepecitabin, Cerivastatim, Cetirizin, Cetrorelix, Cetuximab, Chenodeoxycholsäure, Choriogonadotropin, Ciclosporin, Cidofovir, Cimetidin, Ciprofloxacin, Cisplatin, Cladribin, Clarithromycin, Clavulansäure, Clindamycin, Clobutinol, Clonidin, Clopidogrel, Codein, Coffein, Colestyramin, Cromoglicinsäure, Cotrimoxazol, Cumarin und Cumarinderivate, Darbepoetin, Cysteamin, Cystein, Cytarabin, Cyclophosphamid, Cyproteron, Cytarabin, Daclizumab, Dalfopristin, Danaparoid, Dapiprazol, Darbepoetin, Defepripron, Desipramin, Desirudin, Desloaratadin, Desmopressin, Desogestrel, Desonid, Dexibuprofen, Dexketoprofen, Disoproxil, Diazepam und Diazepamderivate, Didanosin, Dihydralazin, Diltiazem, Dimenhydrinat, Dimethylsulfoxid, Dimeticon, Dipivoxil, Dipyridarnoi, Dolasetron, Domperidon und Domperidanderivate, Donepzil, Dopamin, Doxazosin, Doxorubizin, Doxylamin, Diclofenac, Divalproex, Dronabinol, Drospirenon, Drotrecogin alpha, Dutasterid, Ebastin, Econazol, Efavirenz, Eletripan, Emidastin, Emtricitabin, Enalapril, Encepur, Entacapon, Enfurvirtid, Ephedrin, Epinephrin, Eplerenon, Epoetin und Epoetinderivate, Eprosartan, Eptifibatid, Ertapenem, Esomeprazol, Estrogen und Estrogenderivate, Etanercept, Ethenzamid, Ethinöstradiol, Etofenamat, Etofibrat, Etofyllin, Etonogestrel, Etoposid, Exemestan, Ezetimib, Famciclovir, Famotidin, Faropenandaloxat, Felodipin, Fenofibrat, Fentanyl, Fenticonazol, Fexofenadin, Finasterid, Fluconazol, Fludarabin, Flunarizin, Fluorouracil, Fluoxetin, Flurbiprofen, Flupirtin, Flutamid, Fluvastatin, Follitropin, Fomivirsen, Fondaparinux, Formoterol, Fosfomicin, Frovatriptan, Furosemid, Fusidinsäure, Gadobenat, Galantamin, Gallopamil, Ganciclovir, Ganirelix, Gatifloxacin, Gefitinib, Gemfibrozil, Gemopatrilat, Gentamicin, Gepiron, Gestagen und Gestagenderivate, Ginkgo, Glatiramer, Glibenclamid, Glipizide, Glucagon, Glucitol und Glucitolclerivate, Glucosamin und Glucosaminderivate, Glykosidantibiotika, , Glutathion, Glycerol und Glycerolderivate, Hypothalamushormone, Goserelin, Grepafloxacin, Gyrasehemmer, Guanethidin, Gyrasehemmer, Hämin, Halofantrin, Haloperidol, Harnstoffderivate als orale Antidiabetika, Heparin und Heparinderivate, Herzglykoside, Hyaluronsäure, Hydralazin, Hydrochlorothiazid und Hydrochlorothiazidderivate, Hydroxyomeprazol, Hydroxyzin, Ibritumomab, Ibuprofen, Idarubicin, Ifliximab, Ifosfamid, Iloprost, Imatinib, Imidapril, Imiglucerase, Imipramin, Imiquimod, Imidapril, Indometacin, Indoramin, Infliximab, Insulin, Insulin glargin, Interferone, Irbesartan, Irinotecan, Isoconazol, Isoprenalin, Itraconazol, Ivabradine, Jod und Jodderivate, Johanniskraut, Kaliumsalze, Ketoconazol, Ketoprofen, Ketotifen, Lacidipin, Lamotrigin, Lansoprazol, Laronidase, Latanoprost, Leflunomid, Leminoprazol, Lepirudin, Lercanidipin, Leteprinim, Letrozol, Levacetylmethadol, Levetiracetam, Levocetirizin, Levodopa, Levodrpropicin, Levofloxacin, Levomethadon, Licofelone, Linezolid, Lipinavir, Liponsäure und Liponsäurederivate, Lisinopril, Lisurid, Lofepramin, Lodoxamid, Lomefloxacin, Lomustin, Loperamid, Lopinavir, Loratadin, Lornoxicam, Losartan, Lumefantrin, Lutropine, Magnesiumsalze, Makrolidantibiotika, Mangafodipir, Maprotilin, Mebendazol, Mebeverin, Meclozin, Mefenaminsäure, Mefloquin, Meloxicam, Memantin, Mepindolol, Meprobamat,Meropenem, Mesalazin, Mesuximid, Metamizol, Metformin, Methadon, Methotrexat, Methyl-(5-amino-4-oxopentanoat), Methylnaloxon, Methylnaltrexone, Methylphenidat, Methylprednisolon, Metixen, Metoclopramid, Metoprolol, Metronidazol, Mianserin, Mibefradil, Miconazol, Mifepriston, Miglitol, Miglustad, Milnacipran, Minocyclin, Minoxidil, Misoprostol, Mitomycin, Mizolastin, Modafinil, Moexipril, Montelukast, Moroctocog, Morphinane, Morphin und Morphinderivate, Moxifloxacin, Mutterkornalkaloide, Nalbuphin, Naloxon, Naproxen, Naratriptan, Narcotin, Natamycin, Nateglinid, Nebivolol, Nefazodon, Nelfinavir, Neostigmin, Neramexan, Nevirapin, Nicergolin, Nicethamid, Nifedipin, Nifluminsäure, Nimodipin, Nimorazol, Nimustin, Nesiritid, Nisoldipin, Norfloxacin, Novaminsulfon, Noscapin, Nystatin, Ofloxacin, Oktotride, Olanzapin, Olmesartan, Olsalazin, Oseltamivir, Omapatrilat, Omeprazol, Omoconazol, Ondansetron, Orlistat, Oseltamivir, Oxaceprol, Oxacillin, Oxaliplatin, Oxaprozin, Oxcarbacepin, Oxicodon, Oxiconazol, Oxymetazolin, Palivizumab, Palonosetron, Pantoprazol, Paracetamol, Parecoxib, Paroxetin, Pegaspargase, Peg-Interferon, Pegfilgrastrim, Penciclovir, orale Penicilline, Pentazocin, Pentifyllin, Pentoxifyllin, Peptidantibiotika, Perindopril, Perphenazin, Pethidin, Pflanzenextrakte, Phenazon, Pheniramin, Phenylbuttersäure, Phenytoin, Phenothiazine, Phenserin, Phenylbutazon, Phenytoin, Pimecrolimus, Pimozid, Pindolol, Pioglitazon, Piperazin, Piracetam, Pirenzepin, Piribedil, Pirlindol, Piroxicam, Posaconazole, Pramipexol, Pramlintide, Pravastatin, Prazosin, Procain, Promazin, Propiverin, Propranolol, Propionsaurederivate, Propyphenazon, Prostaglandine, Protionamid, Proxyphyllin, Quetiapin, Quinapril, Quinaprilat, Quinupristin, Ramipril, Ranitidin, Rabeprazol, Raloxifen, Ranolazine, Rasburicase, Reboxetin, Repaclinide, Reproterol, Reserpin, Revofloxacin, Ribavirin, Rifampicin, Riluzole, Rimexolon, Risedronat, Risperidon, Ritonavir, Rituximab, Rivastimen, Risatriptan, Rofecoxib, Ropinirol, Ropivacain, Rosiglitazon, Rotigotine, Roxatidin, Roxithromycin, Ruscogenin, Rosuvastatin, Rutosid und Rutosidderivate, Sabadilla, Salbutamol, Salicylate, Salmeterol, Saperconazole, Schilddrüsenhormone, Scopolamin, Selegilin, Sertaconazol, Sertindol, Sertralin, Sevelamer, Sibutramin, Sildenafil, Silikate, Simvastatin, Sirolimus, Sitosterin, Sotalol, Spagluminsäure, Spaffloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Tadalafil, Taliolol, Talsaclidin, Tamoxifen, Tamsulosin, Tasonermin, Tazaroten, Tegafur, Tegaserod, Telithromycin, Telmisartan, Temoporfin, Temozolomid, Tenatoprazol, Tenecteplase, Teniposid, Tenofovir, Tenoxicam, Teriparatid, Terazosin, Terbinafin, Terbutalin, Terfenadin, Teriparatid, Terlipressin, Tertatolol, Testosteron und Testosteronderivate, Tetracycline, Tetryzolin, Tezosentan, Theobromin, Theophyllin, Theophyllinderivate, Thiamazol, Thiotepa, Thr. Wachstumsfaktoren, Tiagabin, Tiaprid, Tibolon, Ticlopidin, Tilidin, Timolol, Tinidazol, Tioconazol, Tioguanin, Tiotropium, Tioxolon, Tirazetam, Tiropramid, Trofiban, Tizanidin, Tolazolin, Tolbutamid, Tolcapon, Tolnaftat, Tolperison, Tolterodin, Topiramat, Topotecan, Torasemid, Tramadol, Tramazolin, Trandolapril, Tranylcypromin, Trapidil, Trastuzumab, Travoprost, Trazodon, Trepostinil, Triamcinolon und Triamcinolonderivate, Triamteren, Trifluperidol, Trifluridin, Trimetazidine, Trimethoprim, Trimipramin, Tripelennamin, Triprolidin , Trifosfamid, Tromantadin, Trometamol, Tropalpin, Trovafloxacin, Troxerutin, Tulobuterol, Trypsine, Tyramin, Tyrothricin, Urapidil, Ursodeoxycholsäure, Theophyllin Ursodeoxycholsäure, Valaciclovir, Valdecoxib, Valganciclovir, Valproinsäure, Valsartan, Vancomycin, Vardenafil, Vecuroniumchlorid, Venlafaxin, Verapamil, Verteporfin, Vidarabin, Vigabatrin, Viloxazin, Vinblastin, Vincamin, Vincristin, Vindesin, Vinorelbin, Vinpocetin, Viquidil, Vitamin D und Derivate von Vitamin D, Voriconazol, Warfarin, Xantinoinicotinat, Ximelagatran, Xipamid, Zafirlukast, Zalcitabin, Zaleplon, Zanamivir, Zidovudin, Ziprasidon, Zoledronsäure, Zolmitriptan, Zolpidem, Zoplicon, Zotepin und dergleichen.

Die Wirkstoffe können gegebenenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gegebenenfalls können die Arzneiformen auch zwei oder mehrere pharmazeutische Wirkstoffe enthalten.

### BEISPIELE

Allgemeine Versuchdurchführung für alle Beispiele:
**Kernmaterial**: 1000 g Theophyllingranulat 0,3 - 0,8 mm
**Sprühsuspensionen:**

| | Funktion | Material | Rezeptur Menge [g] |
|---|---|---|---|
| 1. | Filmbildner | EUDRAGIT^{®} RS 30 D | 300,0 |
| | | EUDRAGIT^{®} RL 30 D | 33,3 |
| 2. | Weichmacher | Triethylcitrat (TEC) | 20,0 |
| 3. | Trennmittel | Kieselsäure (Syloid 244 FP) | 30,0 |
| 4. | Verdünnungsmittel | Gereinigtes Wasser | 366,7 |
| | | Summe: | 750,0 |
| | | | |
| | | Polymergehalt [Gew.-%]: | 13,3 |
| | | Feststoffgehalt [Gew.-%] | 20,0 |
| | | | |
| | | Polymerauftrag [Gew.-%] | 10,0 |
| | | Gesamtauftrag [Gew.-%] | 15,0 |

**Sprühauftrag:**

| | |
|---|---|
| Gerät: | GPCG 1.1 mit Topspray - Einsatz |
| Düsendurchmesser: | 1,2 mm |
| Düsenabstand zum Produkt: | 10 cm |
| Sprühdruck: | 1,8 bar |
| Zuluftmenge [m³/Std.]: | 77-94 |
| Zulufttemperatur [°C]: | 32-38 |
| Sprührate [g/min x kg]: | 6,0-8,2 |
| Sprühzeit [min]: | 92-94 |
| Produkttemperatur [°C]: | 25,0-29,0 |
| Relative Abluftfeuchte [%]: | 23,5-34,9 |

Nach dem Sprühauftrag wurde 5 min ohne in dem Gerät unter leichtem Wirbeln getrocknet. Anschließend erfolgte der Stabilisierungsschritt über eine Zeitraum von bis zu 2 Std. mit Musternahme nach definierten Zeiten. Alle Muster wurden hinsichtlich Wirkstoffabgabeschwindigkeit untersucht. Zur Bestätigung der Stabilisierung wurde die verbleibende Produktmenge zusätzlich gemäß dem Stand der Technik auf Horden gefüllt und über 24 Stunden bei 40°C im Umluftrockenschrank nachbehandelt (Referenzprobe).

### Bestimmung der Wirkstoffabgabe:

Die Bestimmung der Wirkstofffreigabe wurde analog USP27 - NF22 <724> Drug Release, Extended Release, Apparatus 2, durchgeführt.

Zunächst wurden, die zu untersuchenden Proben 2 h in 800 ml simulierten Magensaft getestet, bevor mit 100 ml Phosphatpuffer auf pH 6.8 umgepuffert wurde.

### Medium 1:

Puffer pH 1.2 (simulierter Magensaft)

| | |
|---|---|
| HCl, 1mol/L | 1000 ml |
| Wasser | 9000 ml |

### Medium 2:

Umpufferung auf pH 6.8 durch Zugabe von

| | |
|---|---|
| Na₃PO₄ | 304 g |
| Wasser | 2000 ml |

### Geräteparameter:

| | |
|---|---|
| Probemenge: | 300 mg |
| Analyen pro Probe: | n = 2 |
| Rühtgeschwindigkeit: | 150 U/min |
| Puffer Volumen: | 800 ml + 100 ml |
| Analysendauer: | 8 Stunden |
| Wellenlänge: | 271 nm |

### Stabiles Wirkstofffreigabeprofil

Die vorliegenden Bespiele dienen lediglich der Verdeutlichung der Erfindung.
Es wurde fiktiv eine Arzneiform angenommen die eine deklarierte Einzeldosis von 300 mg Pellets mit X = 260 mg Wirkstoff, hier Theophyllin, = 100 % Wirkstoff aufweist und zu den deklarierten Zeitpunkten 4 und 8 Stunden Teilmengen T davon freisetzt. Zusätzlich wurden die Messungen auch zu den Zeitpunkten 5 min, 15 min, 30 min, 45 min, 1 h, 1,5 h, 2 h, 2,5 h, 3 h, 3,5, 5 h, 6 h und 7 h ausgeführt. Der Übersichtlichkeit halber sind die einzelnen Messwerte nicht aufgeführt. Sie lagen jedoch bei allen erfindungsgemäßen "stabilen" Arzneiformen auch zu diesen Zeitpunkten innerhalb der definierten Toleranz.

Der Erfolg der Konditionierung kann am Vergleich des Kurvenverlaufs der erfindungsgemäß konditionierten Arzneiform mit der Referenzkonditionierung verfolgt werden. Da diese Darstellung hier durch die Vielzahl der sich überlagernden Kurven und der einzelnen Messpunkte unübersichtlich wäre, werden vereinfacht ein oder mehrere repräsentative Zeitpunkte in Tabellenform wiedergegeben.

### Beispiel 1 (erfindungsgemäß)

Nachbehandlung durch Einsprühen von 240 g Wasser über 30 min mit folgenden Parametern:

| | |
|---|---|
| Sprührate [g/kg x min]: | 7,7-8,2 |
| Zulufttemperatur [°C]: | 75,0 |
| Zuluftmenge [m³/Std.]: | 83-87 |
| Produkttemperatur [°C]: | 49,0-52,0 |
| Relative Feuchte in der Abluft [%]: | 8,1 % -10,1 % |

### Wirkstoffabgabe:

| Messpunkte [min] | Nach Sprühauftrag [%] | Nach 5min Nachtrocknen [%] | Nach 30 min Stabilisierung [%] | Nach 24 h Nachtrocknung [%] |
|---|---|---|---|---|
| 240 | 73,08 | 76,00 | 44,43 | 47,56 |
| 480 | 97,60 | 98,26 | 61,17 | 63,55 |

### Bewertung:

Die Nachbehandlung führt zu einer Verlangsamung der Wirkstoffabgabe und nach 30 min zu einem stabilen Wirkstofffreigabeprofil.

### Beispiel 2 (erfindungsgemäß)

Nachbehandlung durch Einsprühen von 487 g Wasser über 32 min mit folgenden Parametern:

| | |
|---|---|
| Sprührate [g/kg x min]: | 10,1 -14,4 |
| Zulufttemperatur [°C]: | 80 |
| Zuluftmenge [m³/Std.]: | 74 - 76 |
| Produkttemperatur [°C]: | 47,0 - 49,0 |
| Relative Feuchte in der Abluft [%]: | 11,8 - 16,9 |

### Wirkstoffabgabe:

| Messpunkte [min] | Nach Sprühauftrag [%] | Nach 5min Nachtrocknen [%] | Nach 30 min Stabilisierung [%] | Nach 24 h Nachtrocknung [%] |
|---|---|---|---|---|
| 240 | 66,09 | 72,15 | 42,91 | 42,81 |
| 480 | 94,50 | 95,59 | 61,83 | 61,82 |

### Bewertung:

Die Nachbehandlung führt nach 30 min zu einem stabilen Wirkstofffreigabeprofil.

### Beispiel 3 (erfindungsgemäß)

Sprühauftrag gemäß Rezeptur 1 und Verfahren A.
Nachbehandlung durch Einsprühen von 950 g Wasser über 30 min mit folgenden Parametern:

| | |
|---|---|
| Sprührate [g/kg x min]: | 24,7-34,8 |
| Zulufttemperatur [°C]: | 86-90 |
| Zuluftmenge [m³/Std.]: | 81-88 |
| Produkttemperatur [°C]: | 42,0-45,0 |
| Relative Feuchte in der Abluft [%]: | 19,7-29,6 |

### Wirkstoffabgabe:

| Messpunkte [min] | Nach Sprühauftrag [%] | Nach 5min Nachtrocknen [%] | Nach 30 min Stabilisierung [%] | Nach 24 h Nachtrocknung [%] |
|---|---|---|---|---|
| 240 | 75,55 | 67,39 | 53,68 | 50,72 |
| 480 | 96,79 | 91,13 | 73,77 | 70,31 |

### Bewertung:

Die Nachbehandlung führt zu einer Verlangsamung der Wirkstoffabgabe. Ein stabiles Wirkstofffreigabeprofil wird nach 30 min erreicht.

### Beispiel 4 (erfindungsgemäß)

Nachbehandlung durch Einsprühen von 240 g Wasser über 30 min mit folgenden Parametern:

| | |
|---|---|
| Sprührate [g/kg x min]: | 8,5 |
| Zulufttemperatur [°C]: | 75-80 |
| Zuluftmenge [m³/Std.]: | 71-86 |
| Produkttemperatur [°C]: | 47-48 |
| Relative Feuchte in der Abluft [%]: | 8,9 -10,2 |

### Wirkstoffabgabe:

| Messpunkte [min] | Nach SPrühauftrag [%] | Nach 5min Nachtrocknen [%] | Nach 10 min Stabilisierung [%] | Nach 20 min Stabilisierung [%] | Nach 30 min Stabilisierung [%] | Nach 24 h Nachtrocknung [%] |
|---|---|---|---|---|---|---|
| 240 | 98,94 | 86,81 | 75,52 | 63,58 | 58,40 | 60,87 |
| 480 | 99,48 | 98,95 | 95,02 | 83,34 | 76,43 | 78,07 |

### Bewertung:

Die Nachbehandlung führt zu einer stetigen Verlangsamung der Wirkstoffabgabe. Ein stabiles Wirkstofffreigabeprofil wird nach 20 min erreicht.

### Langzeitlagerstabilität (Lagerung in Aluminiumdosen):

Zur Überprüfung des Verhaltens bei längerer Lagerung werden die erfindungsgemäß für 30 min stabilisierten Pellets aus Beispiel 4 für 1 bzw. 3 Monate im Aluminiumdosen eingelagert und anschließend erneut geprüft.

| | | Nach 1 monatiger Lagerung | | | Nach 3 monatiger Lagerung | | |
|---|---|---|---|---|---|---|---|
| Messpunkte [min] | Nach 30 min Stabilisierung | 25°C, 60% rel. Feuchte [%] | 30°C, 60% rel. Feuchte [%] | 40°C, 75% rel. Feuchte [%] | 25°, 60% rel. Feuchte [%] | 30°C, 60% rel. Feuchte [%] | 40°C, 75% rel. Feuchte [%] |
| 240 | 58,40 | 59,67 | 59,69 | 55,73 | 58,75 | 58,86 | 52,23 |
| 480 | 76,43 | 78,37 | 77,65 | 73,19 | 77,22 | 77,03 | 71,31 |

Ergebnis: Die Abweichungen vom vorgesehenen Freigabeprofil liegen innerhalb der vorgesehenen Toleranz. Die Arzneiform ist somit als stabil anzusehen.

### Beispiel 5 (erfindungsgemäß)

Nachbehandlung durch Einsprühen von 240 g einer 10 %igen Suspension von gefällter Kieselsäure (Syloid) in Wasser über 30 min mit folgenden Parametern:
Sprührate [g/kg x min]: 8,2
Zulufttemperatur [°C]: 75
Zuluftmenge [m³/Std.]: 71 - 78
Produkttemperatur [°C]: 42 - 46
Relative Feuchte in der Abluft: 9,2 % -10,1 %

### Wirkstoffabgabe:

| Messpunkte [min] | Nach Sprühauftrag [%] | Nach 5min Nachtrocknen [%] | Nach 30 min Stabilisierung [%] | Nach 24 h Nachtrocknung [%] |
|---|---|---|---|---|
| 240 | 42,46 | 56,66 | 35,83 | 35,59 |
| 480 | 65,69 | 82,70 | 52,06 | 51,82 |

### Bewertung:

Die Nachbehandlung führt zu einer Verlangsamung der Wirkstoffabgabe. Ein stabiles Wirkstofffreigabeprofil wird nach 30 min erreicht.

### Beispiel 6 (erfindungsgemäß)

Sprühauftrag gemäß Rezeptur 1 und Verfahren A.
Nachbehandlung durch Einsprühen von 240 g einer 10 %igen Suspension von gefällter Kieselsäure (Syloid) in Wasser über 30 min mit folgenden Parametern:

| | |
|---|---|
| Sprührate [g/kg x min]: | 8,5 |
| Zulufttemperatur [°C]: | 75 - 80 |
| Zuluftmenge [m³/Std.]: | 75 - 82 |
| Produkttemperatur [°C]: | 47 - 49 |
| Relative Feuchte in der Abluft [%]: | 9,8 - 10,1 |

### Wirkstoffabgabe:

| Messpunkte [min] | Nach Sprühauftrag [%] | Nach 5min Nachtrocknen [%] | Nach 10 min Stabilisierung [%] | Nach 20 min Stabilisierung [%] | Nach 30 min Stabilisierung [%] | Nach 24 h Nachtrocknurn [%] |
|---|---|---|---|---|---|---|
| 240 | 98,58 | 60,20 | 40,73 | 40,62 | 36,67 | 38,9 |
| 480 | 99,55 | 86,90 | 60,17 | 58,33 | 55,08 | 56,6 |

### Bewertung:

Die Nachbehandlung führt zu einer stetigen Verlangsamung der Wirkstoffabgabe. Ein stabiles Wirkstofffreigabeprofil wird nach 10 min erreicht.

### Langzeitlagerstabilität (Lagerung in Aluminiumdosen):

Zur Überprüfung des Verhaltens bei längerer Lagerung werden die erfindungsgemäß für 30 min stabilisierten Pellets aus Beispiel 6 für 1 bzw. 3 Monate im Aluminiumdosen eingelagert und anschließend erneut geprüft.

| | | Nach 1 monatiger Lagerung | | | Nach 3 monatiger Lagerung | | |
|---|---|---|---|---|---|---|---|
| Messpunkte [min] | Nach 30 min Stabilisierung [%] | 25°C, 60% rel. Feuchte [%] | 30°C, 60% rel. Feuchte [%] | 40°C, 75% rel. Feuchte [%] | 25°, 60% rel. Feuchte [%] | 30°C, 60% rel. Feuchte [%] | 40°C, 75% rel. Feuchte [%] |
| 240 | 36,67 | 40,59 | 40,09 | 31,50 | 35,46 | 35,88 | 28,64 |
| 480 | 55,08 | 61,82 | 59,21 | 48,48 | 54,20 | 53,79 | 46,39 |

Ergebnis: Die Abweichungen vom vorgesehenen Freigabeprofil liegen innerhalb der vorgesehenen Toleranz. Die Arzneiform ist somit als stabil anzusehen.

### Beispiel 7 (nicht erfindungsgemäße Konditionierungstemperatur)

Um den Einfluss der Temperatur der Konditionierungstemperatur (entsprechend der Produkttemperatur) und der Luftfeuchtigkeit (Relative Feuchte in der Abluft) zu demonstrieren werden beide außerhalb des erfindungsgemäßen Bereichs eingestellt.

Nachbehandlung durch Einsprühen von 240 g einer 10 %igen Suspension von gefällter Kieselsäure Syloid) in Wasser über 30 min mit folgenden Parametern:

| | |
|---|---|
| Sprührate [g/kg x min]: | 8,5 |
| Zulufttemperatur [°C]: | 30 - 33 |
| Zuluftmenge [m³/Std.]: | 84 - 88 |
| **Produkttemperatur [°C]:** | **24 - 26** |
| **Relative Feuchte in der Abluft [%]:** | **36,9 - 40,2** |

### Wirkstoffabgabe:

| Messpunkte [min] | Nach Sprühauftrag [%] | Nach 5min Nachtrocknen [%] | Nach 10 min Stabilisierung [%] | Nach 20 min Stabilisierung [%] | Nach 30 min Stabilisierung [%] | Nach 24 h Nachtrocknung [%] |
|---|---|---|---|---|---|---|
| 240 | 98,04 | 97,91 | 98,95 | 98,73 | 98,87 | 92,75 |
| 480 | 99,07 | 99,00 | 99,18 | 98,92 | 99,30 | 99,07 |

### Bewertung:

Die Nachbehandlung führt nach 30 min nicht zu einem retardierten Wirkstofffreigabeprofil. Die Permeabilität des Überzuges mit einer Freigabe von ca. 100 % nach 4 bzw. 8 Stunden ist sehr hoch und deutet auf Schäden durch Verklebung in zu großer Feuchte hin.

## Patentansprüche

1. Verfahren zur Herstellung von Arzneiformen mit stabilem Wirkstofffreigabeprofil, wobei die Arzneiformen aufgrund eines Überzuges von Vinyl(co)polymeren eine kontrollierte Freisetzungscharakteristik aufweisen,
**dadurch gekennzeichnet, daß**
die überzogenen Arzneiformen in einem Wirbelschicht-Überzugsgerät oder einem Trommel-Überzugsgerät für mindestens 10 Minuten bis zum Erreichen eines stabilen Wirkstofffreigabeprofils bei einer Temperatur von 30 bis 70 °C konditioniert werden, wobei während der Konditionierung eine Luftfeuchte von 5 bis 30 % eingestellt wird.

2. Verfahren nach Anspruch 1, **dadurch gekennzeichnet, daß** die überzogenen Arzneiformen in einem ersten Herstellungsschritt durch Überziehen von wirkstoffhaltigen Kernen oder Pelletkernen mit Vinyl(co)polymeren im Wirbelschicht-Überzugsgerät oder einem Trommel-Überzugsgerät hergestellt und gegebenenfalls getrocknet werden und in einen zweiten Schritt die Konditionierung der überzogenen Arzneiformen im selben Gerät unmittelbar nach deren Herstellung erfolgt.

3. Verfahren nach Anspruch 1 oder 2, **dadurch gekennzeichnet, daß** die erforderliche Luftfeuchte durch Einsprühen von Wasser eingestellt wird.

4. Verfahren nach einem oder mehreren der Ansprüche 1 bis 3, **dadurch gekennzeichnet, daß** eine wässrige Suspension, enthaltend 0,1 bis 5 Gew.-% Trennmittel, bezogen auf die Suspension eingesprüht wird.

5. Verfahren nach Anspruch 4, **dadurch gekennzeichnet, daß** als Trennmittel Talkum, Magnesiumstearat oder eine Kieselsäure eingesetzt wird.

6. Verfahren nach einem oder mehreren der Ansprüche 1 bis 5, **dadurch gekennzeichnet, daß** die Überzüge der Arzneiformen aus (Meth)acrylat(co)polymeren oder aus Polyvinylacetat oder Derivaten des Polyvinylacetats bestehen.

7. Verfahren nach Anspruch 6, **dadurch gekennzeichnet, daß** die Überzüge der Arzneiformen aus (Meth)acrylat(co)polymeren bestehen, die zu mehr als 95 Gew.-% bis 100 % aus Monomeren mit neutralen Resten polymerisiert sind.

8. Verfahren nach Anspruch 7, **dadurch gekennzeichnet, daß** die (Meth)acrylatcopolymere aus 20 bis 40 Gew.-% Ethylacrylat und 60 bis 80 Gew.-% Methylmethacrylat polymerisiert sind.

9. Verfahren nach Anspruch 6, **dadurch gekennzeichnet, daß** die Überzüge der Arzneiformen aus kationischen (Meth)acrylat(co)polymeren bestehen.

10. Verfahren nach Anspruch 9, **dadurch gekennzeichnet, daß** die Überzüge der Arzneiformen aus (Meth)acrylatcopolymeren bestehen, die quaternäre Aminogruppen enthalten.

11. Verfahren nach Anspruch 10, **dadurch gekennzeichnet, daß** die Überzüge der Arzneiformen aus (Meth)acrylatcopolymeren bestehen, die aus radikalisch polymerisierten Einheiten von 50 - 70 Gew.-% Methylmethacrylat, 20 - 40 Gew.-% Ethylacrylat und 12-2 Gew.-% 2-Trimethylammoniumethylmethacrylat-Chlorid aufgebaut sind.

12. Verfahren nach Anspruch 9, **dadurch gekennzeichnet, daß** die Überzüge der Arzneiformen aus (Meth)acrylatcopolymeren bestehen, die tertiäre Aminogruppen enthalten.

13. Verfahren nach Anspruch 12, **dadurch gekennzeichnet, daß** die Überzüge der Arzneiformen aus (Meth)acrylatcopolymeren bestehen, die aus 20 - 30 Gew.-% Methylmethacrylat, 20 - 30 Gew.-% Butylmethacrylat und 60 - 40 Gew.-% Dimethylaminoethylmethacrylat aufgebaut sind.

14. Verfahren nach Anspruch 6, **dadurch gekennzeichnet, daß** die Überzüge der Arzneiformen aus anionischen (Meth)acrylat(co)polymeren bestehen.

15. Verfahren nach Anspruch 14, **dadurch gekennzeichnet, daß** die Überzüge der Arzneiformen aus anionischen (Meth)acrylat(co)polymeren bestehen, die zu 25 bis 95 Gew.-% aus radikalisch polymerisierten C₁-bis C₄-Alkylestern der Acryl- oder der Methacrylsäure und zu 5 bis 75 Gew.-% aus (Meth)acrylat-Monomeren mit einer anionischen Gruppe im Alkylrest bestehen.

## Claims

1. A method for producing pharmaceutical forms having a stable active ingredient release profile, the pharmaceutical forms having controlled release characteristics on account of a coating of vinyl (co)polymers,
**characterized in that**
the coated pharmaceutical forms are conditioned at a temperature of 30 to 70°C in a fluidized bed coating apparatus or a drum coating apparatus for at least 10 minutes until achievement of a stable active ingredient release profile, an atmospheric humidity of 5 to 30% being set during the conditioning.

2. The method as claimed in claim 1, **characterized in that** the coated pharmaceutical forms are produced in a first production step by coating active ingredient-containing cores or pellet cores with vinyl (co)polymers in a fluidized bed coating apparatus or a drum coating apparatus and optionally drying and in a second step carrying out the conditioning of the coated pharmaceutical forms in the same apparatus immediately after their production.

3. The method as claimed in claim 1 or 2,
**characterized in that** the necessary atmospheric humidity is adjusted by spraying in water.

4. The method as claimed in one or more of claims 1 to 3, **characterized in that** an aqueous suspension comprising 0.1 to 5% by weight of release agent, based on the suspension, is sprayed in.

5. The method as claimed in claim 4, **characterized in that** the release agent employed is talc, magnesium stearate or a silicic acid.

6. The method as claimed in one or more of claims 1 to 5, **characterized in that** the coatings of the pharmaceutical forms consist of (meth)acrylate (co)polymers or of polyvinyl acetate or derivatives of polyvinyl acetate.

7. The method as claimed in claim 6, **characterized in that** the coatings of the pharmaceutical forms consist of (meth)acrylate (co)polymers which are polymerized to more than 95% by weight to 100% from monomers containing neutral radicals.

8. The method as claimed in claim 7, **characterized in that** the (meth)acrylate copolymers are polymerized from 20 to 40% by weight of ethyl acrylate and 60 to 80% by weight of methyl methacrylate.

9. The method as claimed in claim 6, **characterized in that** the coatings of the pharmaceutical forms consist of cationic (meth)acrylate copolymers.

10. The method as claimed in claim 9, **characterized in that** the coatings of the pharmaceutical forms consist of (meth)acrylate copolymers which contain quaternary amino groups.

11. The method as claimed in claim 10, **characterized in that** the coatings of the pharmaceutical forms consist of (meth)acrylate copolymers which are synthesized from free radical-polymerized units of 50 - 70% by weight of methyl methacrylate, 20 - 40% by weight of ethyl acrylate and 12 - 2% by weight of 2-trimethylammonium ethyl methacrylate chloride.

12. The method as claimed in claim 9, **characterized in that** the coatings of the pharmaceutical forms consist of (meth)acrylate copolymers which contain tertiary amino groups.

13. The method as claimed in claim 12, **characterized in that** the coatings of the pharmaceutical forms consist of (meth)acrylate copolymers which are synthesized from 20 - 30% by weight of methyl methacrylate, 20 - 30% by weight of butyl methacrylate and 60 - 40% by weight of dimethylaminoethyl methacrylate.

14. The method as claimed in claim 6, **characterized in that** the coatings of the pharmaceutical forms consist of anionic (meth)acrylate (co)polymers.

15. The method as claimed in claim 14, **characterized in that** the coatings of the pharmaceutical forms consist of anionic (meth)acrylate (co)polymers which consist to 25 to 95% by weight of free radical-polymerized C₁- to C₄-alkyl esters of acrylic or methacrylic acid and to 5 to 75% by weight of (meth)acrylate monomers containing an anionic group in the alkyl radical.

## Revendications

1. Procédé de préparation de formes galéniques à profil stable de libération du principe actif, dans lequel les formes galéniques présentent, en raison d'un enrobage de (co)polymères vinyliques, une caractéristique de libération contrôlée,
**caractérisé en ce que**
les formes galéniques enrobées sont conditionnées dans un appareil d'enrobage à lit fluidisé ou un appareil d'enrobage à tambour pendant au moins 10 minutes jusqu'à l'obtention d'un profil stable de libération du principe actif à une température de 30 à 70°C, pendant le conditionnement, une humidité de l'air de 5 à 30% étant réglée.

2. Procédé selon la revendication 1, **caractérisé en ce que** les formes galéniques enrobées sont préparées lors d'une première étape de préparation par enrobage de noyaux ou de noyaux de comprimés contenant le principe actif avec les (co)polymères vinyliques dans un appareil d'enrobage à lit fluidisé ou un appareil d'enrobage à tambour et sont le cas échéant séchées et, lors d'une deuxième étape a lieu le conditionnement des formes galéniques enrobées dans le même appareil directement après la préparation de celles-ci.

3. Procédé selon la revendication 1 ou 2, **caractérisé en ce que** l'humidité de l'air nécessaire est réglée par injection d'eau.

4. Procédé selon l'une ou plusieurs des revendications 1 à 3, **caractérisé en ce qu'**une suspension aqueuse, contenant de 0,1 à 5% en poids d'un agent de séparation, sur base de la suspension, est pulvérisée.

5. Procédé selon la revendication 4, **caractérisé en ce qu'**on utilise comme agent de séparation du talc, du stéarate de magnésium ou un acide silicique.

6. Procédé selon l'une ou plusieurs des revendications 1 à 5, **caractérisé en ce que** les enrobages des formes galéniques sont composés de (co)polymères de (méth)acrylate ou de polyvinylacétate ou de dérivés du polyvinylacétate.

7. Procédé selon la revendication 6, **caractérisé en ce que** les enrobages des formes galéniques sont composés de (co)polymères de (méth)acrylate qui sont polymérisés à plus de 95% à 100% de monomères avec des radicaux neutres.

8. Procédé selon la revendication 7, **caractérisé en ce que** les copolymères de (méth)acrylate composés de 20 à 40% en poids d'éthylacrylate et de 60 à 80% en poids de méthylméthacrylate sont polymérisés.

9. Procédé selon la revendication 6, **caractérisé en ce que** les enrobages des formes galéniques sont composés de (co)polymères de (méth)acrylate.

10. Procédé selon la revendication 9, **caractérisé en ce que** les enrobages des formes galéniques sont composés de copolymères de (méth)acrylate contenant des groupes amino quaternaires.

11. Procédé selon la revendication 10, **caractérisé en ce que** les enrobages des formes galéniques sont composés de copolymères de (méth)acrylate, constitués à partir de motifs polymérisés par voie radicalaire de 50 à 70% en poids de méthylméthacrylate, de 20 à 40% en poids d'éthylacrylate et de 12 à 2% en poids de chlorure de 2-triméthylammoniuméthylméthacrylate.

12. Procédé selon la revendication 9, **caractérisé en ce que** les enrobages des formes galéniques sont composés de copolymères de (méth)acrylate contenant des groupes amino tertiaires.

13. Procédé selon la revendication 12, **caractérisé en ce que** les enrobages des formes galéniques sont composés de copolymères de (méth)acrylate, constitués de 20 à 30% en poids de méthylméthacrylate, de 20 à 30% en poids de butylméthacrylate et de 60 à 40% en poids de chlorure de diméthylaminoéthylméthacrylate.

14. Procédé selon la revendication 6, **caractérisé en ce que** les enrobages des formes galéniques sont composés de (co)polymères de (méth)acrylate anioniques.

15. Procédé selon la revendication 14, **caractérisé en ce que** les enrobages des formes galéniques sont composés de (co) polymères de (méth)acrylate anioniques, constitués de 25 à 95% en poids d'alkylesters d'acide acrylique ou méthacrylique en C₁-C₄ polymérisés par voie radicalaire et de 5 à 75% en poids de monomères de (méth)acrylate avec un groupe anionique dans le radical alkyle.
